(19) Europäisches Patentamt
European Patent Office
Office européen des brevets

(11) Publication number: **0 463 470 A2**

(12) **EUROPEAN PATENT APPLICATION**

(21) Application number: **91109599.0**

(51) Int. Cl.[5]: **C07D 307/30**, C07D 317/26

(22) Date of filing: **12.06.91**

(30) Priority: **22.06.90 US 543015**

(43) Date of publication of application:
**02.01.92 Bulletin 92/01**

(84) Designated Contracting States:
**AT BE CH DE DK FR GB IT LI NL**

(71) Applicant: **F. HOFFMANN-LA ROCHE AG**
**Postfach 3255**
**CH-4002 Basel(CH)**

(72) Inventor: **Okabe, Masami**
**39 Colonial Terrace**
**Nutley, N.J. 07110(US)**

(74) Representative: **Grossner, Lutz, Dr. et al**
**Grenzacher Strasse 124 Postfach 3255**
**CH-4002 Basel(CH)**

(54) **Process for the preparation of a fluorotetrahydrofuranone derivative.**

(57) (5S)-3-Fluoro-tetrahydro-5-[(hydroxy)methyl] -2(3H)-furanone, a known intermediate for the manufacture of fluorodideoxytidine, is prepared from starting materials that are derived from D-pentoses, particularly D-xylose.

EP 0 463 470 A2

(5S)-3-Fluorotetrahydro-5-[(hydroxy)methyl]-2(3H)-furanone is an important intermediate compound in the production of 2'-fluoro-2',3'-dideoxynucleosides such as 2'-fluoro-2',3'-dideoxycytidine (F-ddC) which has been shown to be an effective anti-retroviral agent. However, the prior art process to produce F-ddC requires the use of toxic chemicals such as hydrogen fluoride-triethylamine complex and tributyltin hydride and the use of ribose which is expensive.

The present invention relates to a new process for the synthesis of (5S)-3-fluorotetrahydro-5-[(hydroxy)-methyl]-2(3H)-furanone. The process of the present invention comprises

a) subjecting a compound having the formula

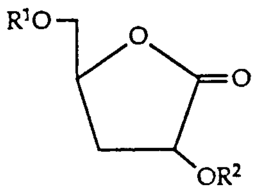

I

wherein $R^1$ and $R^2$ are protecting groups;

to conditions which remove $R^1$ and $R^2$ and that open the lactone ring;

b) converting the carboxylic acid group of the 2,4,5-trihydroxypentanoic acid produced in step (a) to an ester;

c) protecting hydroxy groups present at the carbon 4 and 5 positions of that ester to produce a 4,5-protected dihydroxy 2-hydroxypentanoic acid ester;

d) replacing the 2-hydroxy group with a fluorine atom to produce a 2-fluoro-4,5-protected dihydroxypentanoic acid ester and

e) removing protecting and ester groups from the 2-fluoro-4,5-protected dihydroxypentanoic acid ester and cyclizing the 2-fluoro-4,5-dihydroxypentanoic acid obtained to form (5S)-3-fluorotetrahydro-5-[-(hydroxy)-methyl]-2(3H)-furanone.

In the process of the present invention many different types of protecting groups can be used to protect the appropriate functional groups as is illustrated in "Protective Groups in Organic Synthesis" by Theodora W.Green (John Wiley and Sons).

Preferred protecting groups $R^1$ and $R^2$ are tetrahydropyranyl, trialkylsilyl and, particularly, acyl such as acetyl.

The present invention is also concerned with novel intermediates produced by the process of the present invention: 2,2-dimethyl-1,3-dioxolane-4-(α-hydroxypropanoic acid) methyl ester, 2,2-dimethyl-1,3-dioxolane-4-[α-(methanesulfonyl)oxypropanoic acid] methyl ester and 2,2-dimethyl-1,3-dioxolane-4-(α-fluoropropanoic acid) methyl ester.

In the present application the following abreviations are used for the compounds as indicated below:

| | |
|---|---|
| p-TsOH = | p-toluenesulfonic acid |
| MsCl = | methanesulfonyl chloride |
| TMS = | trimethylsilyl group |
| HMDS = | hexamethyldisilazane |
| DIBAL = | diisobutylaluminium hydride |
| EtoAc = | ethyl acetate |
| NaoAc = | sodium acaetate |

In the preferred method for producing (5S)-3-fluorotetrahydro-5-[(hydroxy)methyl]-2(3H)-furanone according to the present invention, di-O-acetyl-3-deoxy-D-arabino-1,4-lactone is used as the compound of formula I Di-O-acetyl-3-deoxy-D-arabino-1,4-lactone can be prepared from D-xylose. D-xylose is oxidized with bromine followed by acetylation which produces crystalline tri-O-acetyl-D-xylonolactone. Tri-O-acetyl-D-xylonolactone is hydrogenolyzed in ethyl acetate and triethylamine using Raney-Ni as catalyst to afford di-O-acetyl-3-deoxy-D-arabino-1,4-lactone. Another D-pentose, such as arabinose, ribose, or lyxose, can be used to prepare the starting material of formula I [See K.Bock, I.Lundt, and C.Pedersen, Acta Chem. Scand., B35, 155 (1981)].

In a preferred aspect of this invention, di-O-acetyl-3-deoxy-D-arabino-1,4-lactone is treated with 2,2-dimethoxypropane and methanol in the presence of a catalytic amount of p-TsOH monohydrate producing (S,S)-2,2-dimethyl-1,3-dioxolane-4-(α-hydroxypropanoic acid) methyl ester. (S,S)-2,2-Dimethyl-1,3-dioxolane-4-(α-hydroxypropanoic acid) methyl ester is converted to (S,S)-2,2-dimethyl-1,3-dioxolane-4-[α-(methanesulfonyl)oxypropanoic acid] methyl ester. Displacement of the methanesulfonyloxy group by fluorine is effected by treatment with KF in acetamide-toluene to produce (4S-rac)-2,2-dimethyl-1,3-dioxolane-4-(α-fluoropropanoic acid) methyl ester as a mixture of the C-2 epimers (syn : anti = 8-9 : 1) in 55.4% yield from (S,S)-2,2-dimethyl-1,3-dioxolane-4-(α-hydroxypropanoic acid) methyl ester.

The desired anti-isomer (S,S)-2,2-dimethyl-1,3-dioxolane-4-(α-fluoropropanoic acid) methyl ester is enriched (syn : anti = 3 : 5) by treating (4S-rac)-2,2-dimethyl-1,3-dioxolane-4-(α-fluoropropanoic acid) methyl ester with NaOMe (5-7 mol%) in toluene. Further enrichment of the anti-isomer is achieved by fractional distillation using a 4 ft. Goodloe column and a splitter. The early fractions contain the syn-isomer as a major component, and this is isomerized to the anti-enriched material by NaOMe. The anti-isomer (S,S)-2,2-dimethyl-1,3-dioxolane-4-(α-fluoropropanoic acid) methyl ester is thus obtained from D-xylose.

The anti-isomer (S,S)-2,2-dimethyl-1,3-dioxolane-4-(α-fluoropropanoic acid) methyl ester is lactonized to (3S-cis)-3-fluorotetrahydro-5-[(hydroxy)methyl]-2(3H)-furanone on treatment with 2.5 mol% of IN HCl (ca. 1.5eq of water) under reduced pressure to give (3S-cis)-3-fluorotetrahydro-5-[(hydroxy)methyl]-2(3H)-furanone.

This compound can be converted to F-ddC e.g. as described below:

(3S-cis)-3-fluorotetrahydro-5(hydroxymethyl) -2(3H)-furanone is protected as a triphenylmethyl eter. The corresponding dimethylhexylsilyl ether can also be prepared and converted to F-ddC in comparable yield. The corresponding ethoxyethyl ether, and t-butyldimethylsilyl ether can also b converted to F-ddC, however, in lower yields.

(3S-cis)-3-fluorotetrahydro -5-[[[(triphenyl)methyl]oxy]methyl]-2(3H)-furanone is reduced by DIBAL in $CH_2Cl_2$ to give [2(R,S),3S-cis]-3-fluorotetrahydro -5-[[[(triphenyl)methyl]oxy]methyl]-2-furanol as a mixture of anomers ($\beta/\alpha$ >5), which is converted to (2R,3S,4S)-2-chloro-3-fluorotetrahydro -5-[[[(triphenyl)methyl]oxy]-methyl]furan by reaction with mesyl chloride. The coupling of (2R,3S,4S)-2-chloro-3-fluorotetrahydro -5-[[[-(triphenyl)methyl]oxy]methyl]furan with bis-TMS-cytosine gives an anomeric mixture of nucleosides ($\alpha : \beta$ = 1 : 5). This anomer ratio is significantly improved by using bis-TMS-N-acetylcytosine ($\alpha : \beta$ = 1 : >15). The N-acetyl group is partially removed during the reaction. The protecting group at the 5'-position and remaining N-acetyl group are removed by conc. HCl in methanol-dichloromethane to afford the hydrochloride salt of F-ddC which on treatment with a basic ion exchange resin produces F-ddC.

The present invention can be more fully described by the following Examples. Examples 1 and 2 describe the preparation of the compound of formula I.

## Example 1

300 g of D-xylose was dissolved in 800 ml of water. After cooling with an ice-water bath, 340 g of anhydrous $K_2CO_3$ was added in portions, keeping the temperature below 25° C. Then, 120 ml of bromine was added dropwise over 2 hours, keeping the temperature between 4-11° C. After stirring at that temperature for 30 minutes, the mixture was stirred at room temperature overnight. The reaction was quenched by the dropwise addition of 20 ml of 95-97% formic acid over 20 minutes. After stirring for 30 minutes, the mixture was concentrated at 50° C under reduced pressure. The residual water was removed azeotropically with 200 ml of acetic acid at 50° C under reduced pressure. The residue was then added to 400 ml of warm acetic acid. After the mixture was heated to 50° C, 1.8 l of acetic anhydride was added dropwise over 2 hours, keeping the temperature between 50-55° C. After the mixture was stirred at 50° C overnight, the salt was removed by filtration and washed with 400 ml of warm acetic acid. The combined filtrate and washings were concentrated at 50° C under reduced pressure. The residue was dissolved in 1 l of $CH_2Cl_2$ and 1 l of water. The resulting mixture was neutralized by the addition of ca. 170 g of powder $Na_2CO_3$. Then the mixture was diluted with 500 ml of $CH_2Cl_2$ and 1 l of water and the organic layer was separated. The aqueous layer was extracted with 100 ml of $CH_2Cl_2$. The combined extracts were washed with 2 x 1 = 2 l of 1:1 water/saturated aqueous $NaHCO_3$, dried over $Na_2SO_4$, and concentrated. The residue was dissolved in 250 ml of EtOAc and then diluted with 250 ml of hexane. After one day at room temperature, the crystalline material was filtered, washed with 600 ml of EtOAc-hexane (1:2), and dried at 70° C under high vacuum to afford 357 g (65.1%) of tri-O-aceyl-D-xylono-1,4-lactone; mp 97-98° C.

## Example 2

A mixture of 27 g of Raney-Ni (freshly prepared), 274 g of tri-O-acetyl-D-xylono-1,4-lactone dissolved in 2.5 l of EtOAc, and 220 ml of triethylamine was immediately pressurized with 68 bar of hydrogen, then warmed to 30° C, and shaken at that temperature for 24 hours. The catalyst was removed by filtration and washed with a total of 500 ml of EtOAc. The combined filtrate and washings were washed with 1 l of water, and the aqueous layer was back-extracted with 100 ml of EtOAc. The combined extracts were washed with 500 ml of IN HCl, and the aqueous layer was back-extracted with 100 ml of EtOAc. The combined extracts

were then washed with 500 ml of saturated $NaHCO_3$ and 500 ml of brine, dried over $Na_2SO_4$, concentrated, and dried under high vacuum to afford 199 g (92.0%) of di-O-acetyl-3-deoxy-D-arabino-1,4-lactone.

This crude di-O-acetyl-3-deoxy-D-arabino-1,4-lactone was used for the next step without further purification. An analytical sample was prepared by recrystallization from EtOAc-hexane; mp 69-70 °C.

Example 3

A mixture of 199 g of di-O-acetyl-3-deoxy-D-arabino-1,4-lactone, 400 ml of methanol (water content 0.1%), 200 ml of 2,2-dimethoxypropane, and 19 g (0.10 mol) of p-toluenesulfonic acid monohydrate was stirred at 30 °C overnight. 200 ml of 2,2-dimethoxypropane was added and the mixture was stirred at that temperature for 90 minutes. The reaction was quenched with 10 g of NaOAc followed by 5 g of $NaHCO_3$. After stirring for 30 minutes, the mixture was concentrated at 25 °C under reduced pressure. The residue was dissolved in 1 l of EtOAc and 250 ml of saturated $NaHCO_3$. The aqueous layer was back-extracted with 100 ml of EtOAc. The combined organic layers were washed with 100 ml of brine, dried over $Na_2SO_4$, concentrated, and dried under high vacuum to afford 166 g (88.5%) of (S,S)-2,2-dimethyl-1,3-dioxolane-4-(α-hydroxypropanoic acid)methyl ester.

This crude alcohol was used for the next step without further purification. An analytical sample was prepared by distillation; bp 95-105 °C/0.4 hPa.

Example 4

To a mixture of 286 g of (S,S)-2,2-dimethyl-1,3-dioxolane-4-(α-hydroxypropanoic acid) methyl ester, 1.2 l of $CH_2Cl_2$ (water content 0.004%), and 293 ml of $Et_3N$ (water content 0.1%) 141 ml of mesyl chloride was added dropwise over 40 minutes, keeping the temperature below 10 °C. Then the reaction mixture was allowed to warm to 18 °C over 90 minutes. The mixture was washed with 2 x 500 ml = 1 l of water. The aqueous washings were extracted with 100 ml of $CH_2Cl_2$. The combined organic layers were washed with 200 ml of brine, dried over $Na_2SO_4$, concentrated, and dried under high vacuum to afford 395 g (100%) of crude (S,S*)-2,2-dimethyl-1,3-dioxolane-4[α-(methanesulfonyl) oxypropanoic acid] methyl ester. This crude (S,S*)-2,2-dimethyl-1,3-dioxolane-4-[α-(methanesulfonyl) oxypropanoic acid] methyl ester was used for the next step without further pufification.

Example 5

A mixture of 1.0 kg of acetamide, 150 g of spray-dried KF, and 200 ml of toluene (water content 0.01%) was refluxed for 4.5 hours to remove water. Then 395 g of the crude (S,S*)-2,2-dimethyl-1,3-dioxolane-4-[α-(methanesulfonyl) oxypropanoic acid] methyl ester prepared in Example 4 was added with the aid of 200 ml of toluene (water content 0.01%). The mixture was refluxed for 2.5 hours. After the mixture was cooled to 70 °C, 500 ml of toluene and 1.5 l of water were added. The aqueous layer was extracted with 3 x 500 ml = 1.5 l of toluene. The combined organic layers were washed with 500 ml of brine, dried over $Na_2SO_4$, and concentrated. The residue was distilled under high vacuum to give 160 g (55.4% yield) of (4S-rac)-2,2-dimethyl-1,3-dioxolane -4-(α-fluoropropanoic acid) methyl ester; bp 64 °C/0.27 hPa, 92.9% pure.

The product was subjected to epimerization at C-2 to afford anti-enriched material. Since there is a small difference in the boiling points of the syn- and anti-isomers it is possible to obtain pure anti-isomer (S,S)-2,2-dimethyl-1,3-dioxolane-4-(αfluoropropenoic acid) methyl ester by fractional distillation.

To 160 g of (4S-rac)-2,2-dimethyl-1,3-dioxolane-4-(α-fluoropropanoic acid) methyl ester obtained as described above and 776 ml of toluene (water content 0.01%) 10 ml of 25% NaOMe in methanol was added dropwise. After stirring at room temperature for 3 hours, the diastereomeric excess of the anti-isomer reached 24%. After stirring of another hour, the reaction was quen- ched with 2.5 ml for acetic acid. Then the mixture was washed with 2 x 50 ml = 100 ml of brine, dried over $Na_2SO_4$, and concentrated.

The residue was distilled overnight under reduced pressure (1.6 hPa) through a 4 ft. Goodloe column. Reflux ratio was set to 20. There was obtained

Fraction 1: 65.8 g, bp 68 °C, 91.8% pure, 15.1% de (degree of enrichment) of syn-isomer.

To 65.8 g of (4S-rac)-2,2-dimethyl-1,3-dioxolane-4-(α-fluoropropanoic acid) methyl ester (Fraction 1) and 391 ml of toluene (water content 0.01%). 4 ml of 25% NaOMe in methanol was added dropwise. After stirring at room temperature for 2 hours, the reaction was quenched with 1.0 ml (17.5 mol) of acetic acid. Then the mixture was washed with 2 x 25 ml = 50 ml of brine, dried over $Na_2SO_4$, and concentrated to afford 25% de of the anti-isomer.

This was transferred back to the distillation pot and distilled overnight under a reduced pressure (0.93

hPa). Reflux ratio was set to 30. There was obtained

Fraction 2: 47.1 g, bp 62 °C, 87.5% pure, 0.0% de.

This fraction was treated with base as above and transferred back to the distillation pot and distilled under a reduced pressure (2.0 mm Hg). Reflux ratio was set to 30. There were obtained

Fraction 3: 25.7 g, bp 77 °C, 80.8% pure, 9.7% de of anti-isomer.

Fraction 4: 43.3 g, bp 77 °C, 98.9% pure, 25.3% de of anti-isomer.

Fraction 5: 0.2 g, bp 77 °C, 100.0% pure, 39.6% de of anti-isomer.

The distillation was halted at this point. The material in the pot (56 g, 90.8% pure, 84.4% de of anti-isomer) was collected and distilled through a 10 cm-Vigreux column under a reduced pressure (0.4 hPa).

Fraction 6: 52.7 g, bp 65 °C, 93.8% pure, 83.5% de of anti-isomer.

Fraction 7: 1.1 g, bp >65 °C, 46.9% pure, 91.2% de discarded.

The Goodloe column was washed with acetone. The washing was concentrated and saved as Fraction 8.

Fraction 8: 26.8 g, 100.0% pure, 53.6% de of anti-isomer.

Fractions 3-5 and 8 were combined and saved for a future distillation.

96.0 g (60% recovery), ca. 29% de of anti-isomer.

Fraction 6 (52.7 g) was used in the next step; 93.8% pure, 83.5% de of anti-isomer, (S,S*)-2,2-Dimethyl-1,3-dioxolane-4-(α-fluoropropanoic acid)methyl ester.

Example 7

A mixture of 41.2 g of (S,S)-2,2-dimethyl-1,3-dioxolane-4-(α-fluoropropanoic acid) methyl ester (83.5% de of anti-isomer) and 5 ml of IN HCl was stirred at 50-55 °C (bath temperature) under atmospheric pressure for 30 minutes and then under a reduced pressure of 60 mmHg overnight. The excess water was removed at 50-55 °C (bath temperature) under high vacuum (0.4 hPa) for 1.5 hour. The crude (3S-cis)-3-fluorotetrahydro-5-[(hydroxy)methyl]-2(3H)-furanone can be used for conversion to F-ddC as described above. An analytical sample was prepared by distillation; bp 150 °C (bath)/0.8 hPa).

**Claims**

**1.** A process for producing (5S)-3-fluorotetrahydro-5-[(hydroxy)methyl]-2(3H)-furanone comprising:

a) subjecting a compound having the formula

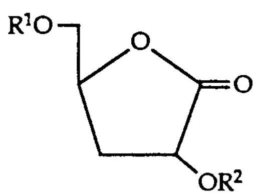

I

wherein $R^1$ and $R^2$ are protecting groups;

to conditions which remove $R^1$ and $R^2$ and that open the lactone ring;

b) converting the carboxylic acid group of the 2,4,5-trihydroxypentanoic acid produced in step (a) to an ester;

c) protecting hydroxy groups present at the carbon 4 and 5 positions of that ester to produce a 4,5-protected dihydroxy 2-hydroxypentanoic acid ester;

d) replacing the 2-hydroxy group with a fluorine atom to produce a 2-fluoro-4,5-protected dihydroxypentanoic acid ester and

e) removing protecting and ester groups from the 2-fluoro-4,5-protected dihydroxypentanoic acid ester and cyclizing the 2-fluoro-4,5-dihydroxypentanoic acid obtained to form (5S)-3-fluorotetrahydro-5-[(hydroxy)-methyl]-2(3H)-furanone.

**2.** A process as in claim 1, wherein $R^1$ and $R^2$ are tetrahydropyranyl, trialkylsilyl, or acyl groups.

**3.** The process as in claim 1 wherein $R^1$ and $R^2$ are acetyl.

**4.** A process as in claim 1, wherein the 4,5-protected 2-hydroxypentanoic acid ester is (S,S)-2,2-dimethyl-1,3-dioxolane-4-(α-hydroxypropanoic acid) methyl ester and the 2-fluoro-4,5-protected dihydroxypentanoic acid ester is (4S)-2,2-dimethyl-1,3-dioxolane-4-(α-fluoropropanoic acid) methyl ester.

**5.** A process as in claim 4, further comprising:
epimerizing (4S-rac)-2,2-dimaethyl-1,3-dioxolane-4-(α-fluoropropanoic acid) methyl ester so as to produce (S,S)-2,2-dimethyl-1,3-dioxolane-4-(α-fluoropropanoic acid) methyl ester.

**6.** A process as in any one of claims 1-5 wherein the compound of formula I is di-O-acetyl-3-deoxy-D-arabino-1,4-lactone.

**7.** 2,2-Dimethyl-1,3-dioxolane-4-(α-hydroxypropanoic acid) methyl ester.

**8.** 2,2-Dimethyl-1,3-dioxolane-4-[α-(methanesulfonyl) oxypropanoic acid] methyl ester.

**9.** 2,2-Dimethyl-1,3-dioxolane-4-(α-fluoropropanoic acid) methyl ester.